# EUROPEAN PATENT APPLICATION

(11) **EP 0 950 718 A1**
(43) Date of publication of application: **20.10.1999**
(21) Application number: 98931022.2
(22) Date of filing: 09.07.1998
(51) Int. Cl.: C12Q 1/68, G01N 21/27, C07K 14/00

(54) **METHOD FOR DETECTING DNA WITH PROBE PNA**

(30) Priority: 09.07.1997 JP 18371097; 24.03.1998 JP 7535098; 22.05.1998 JP 14143398
(71) Applicant: DAI NIPPON PRINTING CO., LTD., Tokyo 162-0062 (JP); Karube, Isao, Kawasaki-shi, Kanagawa 216-0002 (JP)
(72) Inventor: KARUBE, Isao, Kawasaki-shi, Kanagawa 216-0002 (JP); SAWATA, Shinya, Tsuchiura-shi, Ibaraki 300-0013 (JP); NAGATA, Ryohei, Dai Nippon Printing Co., Ltd., Tokyo 162-0062 (JP)
(74) Representative: Dost, Wolfgang, Dr.rer.nat., Dipl.-Chem.
(86) International application number: JP9803077
(87) International publication number: WO9902730

(57) **Abstract**

A method for detecting a target nucleotide sequence by a hybridization procedure with an extremely improved detecting sensitivity. This method involves the step of hybridizing the target nucleotide sequence with a PNA which is complementary to the whole target nucleotide sequence or a part thereof and the step of measuring the extent of the hybridization.

## Description

### BACKGROUND OF THE INVENTION

### Field of the Invention

The present invention relates to a method for detecting a DNA using a PNA (peptide nucleic acid) as a probe.

### Background Art

Methods for detecting a target DNA by using a DNA that is complementary to the DNA sequence are well known. The complementary DNA for detecting the target DNA is referred to as "probe DNA" or simply "probe".

Detection of a target DNA by using a probe DNA is used, for example, in detecting pathogenic microorganisms, or in cloning genes. However, several problems remain unsolved.

For example, a decrease in the salt concentration of the hybridization solution results in a decrease in sensitivity of detection of a target DNA. Therefore, it was necessary to carry out the hybridization in a buffer solution of a fixed salt concentration. Generally, the preparation of such buffer solutions is troublesome and has been one of the factors preventing the simplification of this detection method.

### SUMMARY OF THE INVENTION

The inventors have now found that the sensitivity of detecting a target DNA by the hybridization method is remarkably improved by using a probe PNA in place of a probe DNA.

A decrease in sensitivity of detecting a target DNA by the hybridization method could be due to, but not be limited to, the fact that phosphorus atoms or the like in a DNA molecule are negatively charged. More specifically, it is assumed that electric repulsion is generated between the probe DNA and the target DNA when the DNA is charged thus obstructing hybridization of the two DNAs, which results in a decrease in sensitivity of detection. The remarkable improvement mentioned above is considered to be the result of the absence of an electrical repulsion between the target DNA and the probe PNA.

An object of the present invention is to provide a method of detecting a target nucleotide sequence using a probe. The method accoding to the present invention has a remarkably improved sensitivity of detection.

The method of detecting a target nucleotide sequence according to the present invention comprises the steps of hybridizing a target nucleotide sequence with a PNA that is complementary to the whole or a part of the target nucleotide sequence, then measuring the degree of hybridization.

### BRIEF DESCRIPTION OF THE DRAWINGS

Figure 1 shows the chemical structures of DNA and PNA.
Figure 2 shows a surface plasmon resonance biosensor. 7: cartridge block; 71: measuring cell; 72 and 73: passage; 8: light source; 80: incident light; 9: detector; 90: reflecting light; 10: measuring chip.
Figure 3 shows the measuring chip for the surface plasmon resonance biosensor. 1: transparent substrate; 2: metal membrane; 3: organic layer; 4: avidin; 5: biotin; 6: probe PNA.
Figure 4 shows a DNA sequence coding for Type II verotoxin of pathogenic *Escherichia coli* O-157.
Figure 5 shows the relationship between the amount of amplification products and resonance signals when the length of the sample DNA chain is 143 bp. PCR: 10 cycles(●), 20 cycles(▲), 25 cycle (■), 30 cycles (○) and 40 cycles(△).
Figure 6 shows the relationship between the amount of amplification products and resonance signals when the length of the sample DNA chain is 256 bp.
Figure 7 shows the relationship between the amount of amplification products and resonance signals when the length of the sample DNA chain is 284 bp.
Figure 8 shows the relationship between the amount of amplification products and resonance signals when the length of the sample DNA chain is 391 bp.
Figure 9 shows the relationship between the length of the sample DNA chain and resonance signals.
Figure 10 shows a surface plasmon resonance biosensor.

### DETAILED DESCRIPTION OF THE INVENTION

PNA is a substance that imitates DNA. Like DNA, PNA has nucleic acid bases such as adenine, guanine, cytosine, or thymine in the molecule and hybridizes specifically with a nucleotide sequence having complementary nucleic acid bases. Thus, PNA is almost similar to DNA in its functions but completely different from DNA in its structure.

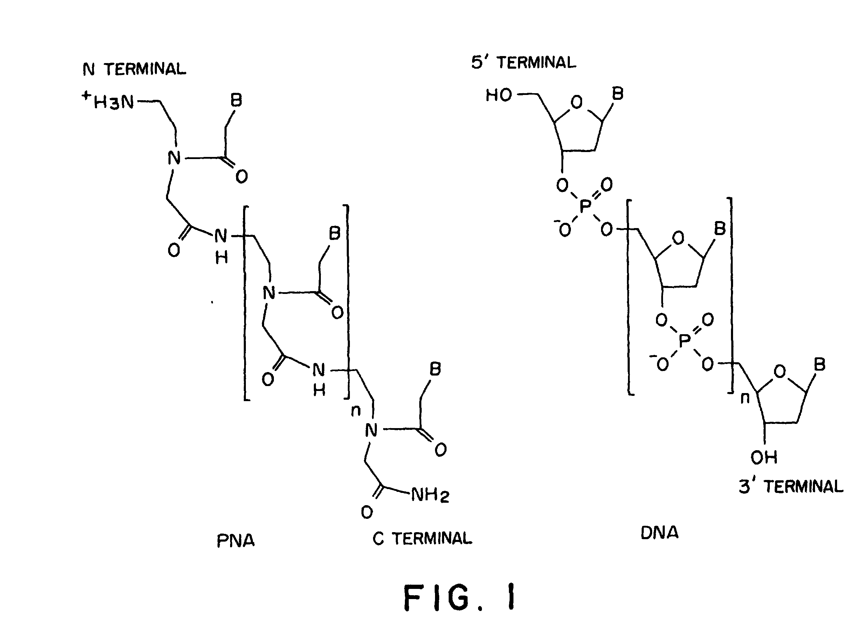

PNA consists of a polyamide backbone structure composed of N-(2-aminoethyl)glycine units (Figure 1) and has no sugar or phosphorus atom in its molecule. Therefore, it is electrically neutral and will not be electrically charged even in the absence of a salt. Nucleic acid bases are bound to the polyamide backbone via methylene carbonyl bonds (Figure 1).

PNA can be synthesized, like peptides, for example, by the Fmoc-type peptide solid phase synthesis method or the tBoc-type peptide solid phase synthesis method. Alternatively, synthesized PNA could be purchased.

The target nucleotide sequence to be detected by the method according to the present invention is not particularly restricted, provided that at least a part of its sequence is known. Examples of the target nucleotide sequence include DNA coding for verotoxin of pathogenic *Escherichia coli*, DNA coding for gp120 (the coating protein of HIV), specific nucleotide sequences (cDNA) of 16SrRNAs of various microorganisms, and a DNA coding for the antibiotic binding protein of methicillin-resistant staphylococcus (MRSA). The target nucleotide sequence may contain impurities. For example, a heat-treated pathogenic *E. coli* preparation can be used without further purification as a test sample in detecting the DNA coding for verotoxin of pathogenic *E. coli*.

In detecting a target nucleotide sequence using a surface plasmone resonance biosensor, it is desirable to first amplify the target nucleotide sequence by a polymerase chain reaction (PCR) method. Conditions for PCR are not particularly restricted. However, significant resonance signals cannot be detected if the concentration of the nucleotide sequence in a sample is too low, while the detection of resonance signals will be reduced if the concentration of the nucleotide sequence in a sample is too high because of interaction between nucleotide sequences. Therefore, 20 to 25 cycles of PCR are preferable.

The method of detecting DNA according to the present invention can be carried out in any mode as long as it is based on the hybridization of a probe PNA and a target nucleotide sequence. An example of a preferred embodiment is the use of a surface plasmon resonance biosensor. The surface plasmon resonance biosensor and measuring chips to be used for detecting a target nucleotide sequence according to the present invention will be explained as follows:

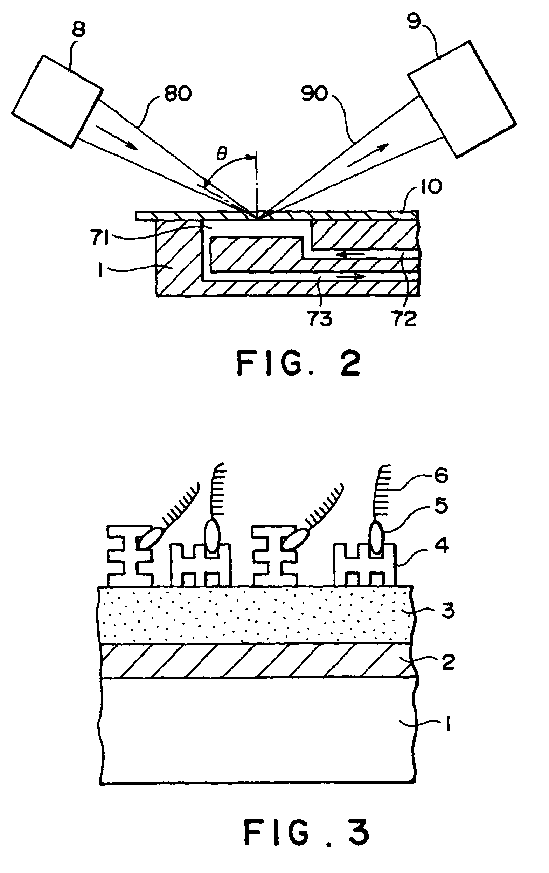

An example of a surface plasmon resonance biosensor used in the present invention is shown in Figure 2. This surface plasmon resonance biosensor has a cartridge block 7, a light source 8 and a detector 9 and is used by placing a measuring chip 10 on which probe PNA 6 is immobilized. Chip 10 is provided on cartridge block 7. The upper side of cartridge block 7 has a hollow and a measuring cell 71 consists of this hollow and measuring chip 10. Measuring cell 71 is communicated with the outside of cartridge block 7 via passages 72 and 73. The sample flows into measuring cell 71 via passage 72 and is discharged after measurement via passage 73.

Monochromatic light (an incident light 80) is irradiated from light source 8 toward the transparent substrate of measuring chip 10. A reflected light 90 which is reflected by a metal membrane set on the reverse side of measuring chip 10 reaches detector 9 which can detect the intensity of reflected light 90.

The biosensor as shown in Figure 2 yields a reflected light intensity curve which forms a trough relative to a given angle of incidence θ. The trough in the reflected light intensity curve is due to surface plasmon resonance. When light is totally reflected at the interface between the transparent substrate and the exterior of measuring chip 10, a surface wave known as an evanescent wave is generated at the interface and a surface wave known as a surface plasmon is also generated on the metal membrane. Resonance occurs when the wave number of these two surface waves coincides, and a part of light energy is consumed to excite the surface plasmon resulting in a decrease in the intensity of the reflected light. The wave number of the surface plasmon is affected by the refractive index of the medium proximate to the surface of the metal membrane. Therefore, when the refractive index of the medium changes due to an interaction between the targeted nucleotide sequence and the probe PNA, a surface plasmon resonance is induced to change the angle of incidence θ. Thus, a change in the concentration of the targeted nucleotide sequence can be perceived by a shift of the trough in the reflected light intensity curve. The change in the angle of incidence θ is called a resonance signal and a change of 10⁻⁴ degree is expressed as 1 RU.

Measuring chip 10 may have a transparent substrate and a metal membrane necessary for surface plasmon resonance and the probe PNA can be immobilized on the metal membrane of the chip. Commercially available measuring chips (for example, a measuring chip for BIAcore 2000, Pharmacia Biosensor, Inc.) may be used. The measuring chip as shown in Figure 3 is preferable. A metal membrane 2 and an organic layer 3 are molded onto a transparent substrate 1. Avidin 4 is immobilized on the organic layer, and probe PNA 6 labeled with biotin is immobilized on avidin 4.

Transparent substrate 1 is not particularly restricted, and can be any substrate used in a measuring chip for a surface plasmon resonance biosensor. Generally, substrates made of materials which are transparent to a laser beam, such as glass, poly(ethylene terephthalate) and polycarbonates can be used. A material which is not anisotropic to polarized light and which can be easily processed is desirable. The thickness of the substrate can be about 0.1 to 20 mm.

Metal membrane 2 is not particularly restricted provided it can induce surface plasmon resonance. Examples of the metal to be used for this metal membrane include gold, silver, copper, aluminum and platinum. They can be used alone or in combination. Furthermore, for better adhesion to the transparent substrate, an auxiliary layer may be set between transparent substrate 1 and the layer made of gold, silver or the like.

The thickness of metal membrane 2 is preferably 100 to 2000 angstroms, most preferably 200 to 600 angstroms. When the thickness exceeds 3000 angstroms, surface plasmon phenomena of the medium cannot be sufficiently detected. Furthermore, when an auxiliary layer made of chrome is used, the thickness of the layer is preferably 5 to 50 angstroms.

Metal membrane 2 can be formed by a conventional method such as sputtering, vacuum evaporation, ion plating, electroplating or non-electrolytic plating. The sputtering method is preferable.

Organic layer 3 consists of a substance which can bind both to a metal atom and to an avidin molecule. The thickness of the organic layer is preferably 10 to 200 angstroms, most preferably 10 to 50 angstroms. Furthermore, aside from an avidin-biotin bond, the probe PNA can be immobilized on organic layer 3 using a covalent bond, such as an ester bond or amide bond.

The organic layer 3 can be formed using a silane coupling agent or a compound having a mercapto group and another organic functional group (thiol compound), or using the LB (Langmuir-Blodgett's) technique. A membrane formed by the LB technique binds to the metal membrane weaker than a membrane formed using a silane coupling agent or a thiol compound. However, the LB technique is applicable to a wider range of substances and can form an agglomerated membrane. Therefore, the number of avidin 4 to be bound per unit area can be increased.

Examples of silane coupling agents that can be used to form the organic layer include 3-aminopropyltriethoxysilane,
3-aminopropyltrimethoxysilane,
3-aminopropyldiethoxymethylsilane,
3-(2-aminoethylaminopropyl)trimethoxysilane,
3-(2-aminoethylaminopropyl)dimethoxymethylsilane,
3-mercaptopropyltrimethoxysilane and
dimethoxy-3-mercaptopropylmethylsilane.

Examples of thiol compounds include:
mercaptoaminomethane, 2-mercapto-1-aminoethane, 3-mercapto-1-aminopropane, 4-mercapto-1-aminobutane, 1,1,1-triamino-2-mercaptoethane, mercaptoacetic acid, 2-mercaptopropionic acid, 3-mercaptobutyric acid, 4-mercaptovaleric acid and 1,1,1-triamino-3-mercaptopropane.

Multi-functional substances having many binding sites with avidin, such as 1,1,1-triamino-2-mercaptoethane and 1,1,1-triamino-3-mercaptopropane, are preferably used. Examples of substrates applicable to the LB technique include 21-aminodocosanoic acid, stearyl amine and polylysine.

Examples of methods for forming the organic layer by a silane coupling agent include the exposure of a metal membrane to saturated vapor of a silane coupling agent for a certain period of time (saturated vapor method), the immersion of a metal membrane into a solution containing a silane coupling agent (immersion method), a spin coater (spin coating method) and a photogravure press (gravure method). The saturated vapor method, immersion method, spin coating method or gravure method can be used to form organic layer 3 using a thiol compound.

Avidin 4 can be immobilized on organic layer 3 by contacting a fixed amount of avidin 4 with organic layer 3 for a fixed period of time. More specifically, transparent substrate 1 with adhered organic layer 3 is positioned on the flow cell type surface plasmon resonance biosensor and a fixed amount of avidin 4 is poured for a fixed period of time.

Examples of methods to immobilize probe PNA 6 labeled with biotin 5 on avidin 4 include the ink jet method and macro dispenser method. The ink jet method has an advantage in that it can precisely eject a drop containing probe PNA 6 onto an extremely small area so that probe PNA 6 to be immobilized can be utilized efficiently. Immobilization can be carried out by positioning a measuring chip onto a flow cell type surface plasmon resonance biosensor and pouring a certain amount of probe PNA 6 for a fixed period of time. This immobilizing method has an advantage that immobilization of avidin 4 and probe PNA 6 can be done consecutively. Labeling the probe PNA 6 with biotin 5 may carried out by extending a PNA strand from biotin by the use of the peptide solid-phase method.

DNA detection using the surface plasmon resonance biosensor is performed by pouring a sample containing the target DNA into a measuring cell of the sensor. The DNA concentration in the sample is preferably 0.1 to 1 µM. Although DNA can be detected by this method in the virtual absence of salt in a sample, a salt concentration of 150 to 300 mM is preferable to detect DNA with high sensitivity.

The target nucleotide sequence may be one or more. Two or more kinds of target nucleotide sequences can be detected by immobilizing multiple numbers of PNAs onto one chip or by providing multiple numbers of chips onto the sensor. Detection of two or more kinds of target nucleotide sequences in this manner will provide better accuracy in detection of the nucleotide sequences.

Whether a sample contains DNA derived from a certain microorganisms can be identified with high accuracy, for example, by immobilizing two or more PNAs complementary to specific DNA of said microorganism. Accuracy can be improved also by including a PNA which does not bind to the target DNA (negative probe) in the DNA sequences being immobilized. Furthermore, by selectively immobilizing a nucleotide sequence, not only the presence or absence of verotoxin in the sample but also the type of toxin, type I or type II, can be determined.

When two or more PNAs are immobilized, the surface plasmon resonance biosensor to be used is preferably of the type in which the measuring chip can freely move in the horizontal direction. Such sensor will enable the measurement of signals of multiple numbers of samples on the chip while leaving the optical system fixed.

### EXAMPLES

### Example 1

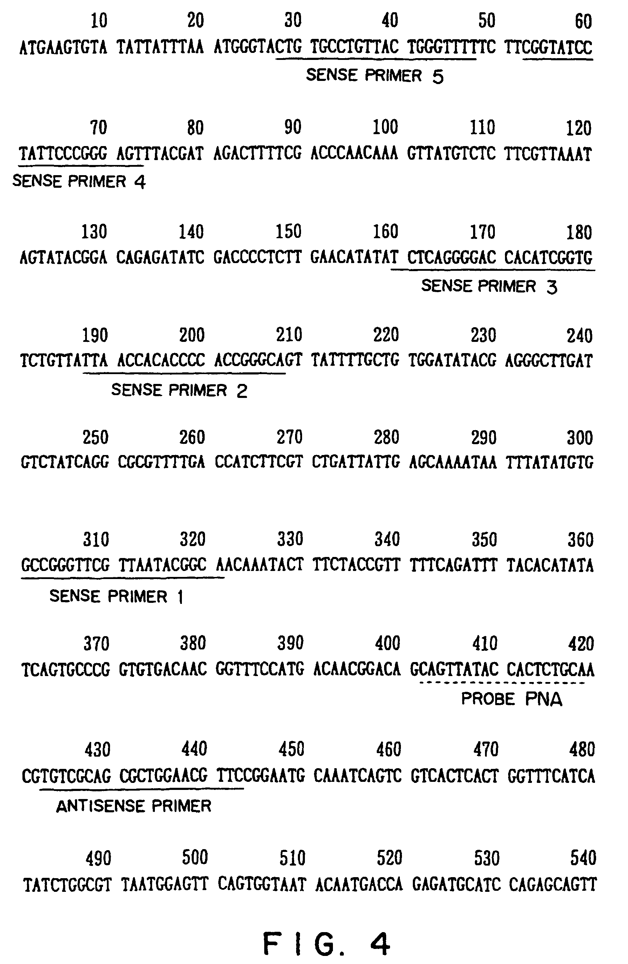

A 5% avidin solution was poured into the measuring cell of a commercial surface plasmon resonance biosensor (BIAcore 2000, Pharmacia Biosensor) at a flow rate of 5 µl/min for 7 minutes to immobilize avidin onto the measuring chip. Meanwhile the following PNA which is complementary to a part of the DNA sequence (SEQ ID NO. 1, Figure 4) coding for type II verotoxin, and has biotin bound at its 5' end was synthesized (synthesized by Japan Perceptive Inc.).
TGCAGAGTGGTATAACTG (SEQ ID NO. 2)
(This sequence is complementary to the DNA sequence of 402-419 of SEQ ID NO. 1.)

A solution containing this probe PNA (10 µM) was poured into the measuring cell of the biosensor at a flow rate of 1 µl/min for 50 minutes to immobilize the probe PNA via avidin onto the measuring chip. The measuring cell was then washed with a 50 mM NaOH solution.

The following single-stranded DNA complementary to the probe PNA was synthesized:
CAGTTATAACCACTCTGCAACG (SEQ ID NO. 3)
(This sequence is identical to the DNA sequence 402-422 of SEQ ID NO. 1.)

Solutions containing this DNA and having the following composition with different salt concentrations were prepared:

**Table 1**

| Composition | Concentration |
|---|---|
| Tris buffer solution (pH 7.5) | 10 mM |
| DNA | 0.1 µM |
| EDTA (pH 8.0) | 10 mM |
| NaCl | 0 to 300 mM |

These solutions with different salt concentrations were poured into the measuring cells at a flow rate of 5 ml/min for 10 minutes and then resonance signals were measured. Results are shown in Table 2.

**Table 2**

| Probe | Salt concentration | | |
|---|---|---|---|
| | 0 mM | 10 mM | 300 mM |
| DNA | Undetectable | 37.6 RU | 253.1 RU |
| PNA | 68.4 RU | 87.4 RU | 157.7 RU |

As shown in Table 2, with the salt concentration of 0 mM, resonance signals were virtually undetectable with the DNA probe, but were detectable with the PNA probe. These results showed that detection sensitivity with a PNA probe was less affected by salt concentration than was the case with a DNA probe. Therefore, the use of a PNA probe enables a target DNA to be detected simply by desalting the PCR products.

### Example 2

The following two sense primers and one antisense primer were synthesized based on the genome DNA of pathogenic *E. coli* O-157 shown in Figure 4.
Sense primer 1: GCCGGGTTCGTTAATACGGCA (SEQ ID NO. 4) (This sequence is identical to the DNA sequence 301-321 of SEQ ID NO. 1)
Sense primer 5: CTGTGCCTGTTACTGGGTTTT (SEQ ID NO. 5) (This sequence is identical to the DNA sequence 28-48 of SEQ ID NO. 1)
Antisense primer 1: GAACGTTCCAGCGCTGCGACA (SEQ ID NO. 6) (This sequence is complementary to the DNA sequence 423-443 of SEQ ID NO. 1)

A solution for PCR having the following components containing these primers and a genome DNA of pathogenic *E. coli* O-157 used as a template was prepared.

**Table 3**

| Component | Concentration |
|---|---|
| Tris buffer solution (pH 8.3) | 25 mM |
| Primer | 1 µM |
| Template DNA | 1% |
| Potassium chloride | 500 mM |
| Magnesium chloride | 15 mM |
| dNTP | 8% |
| Taq polymerase | 0.2% |

After an initial denaturation (95°C, 3 minutes), PCR was performed for 40 cycles of denaturation (61°C, 1 minute), annealing (72°C, 1 minute) and elongation (94°C, 1 minute).

Two kinds of PCR amplification products containing 143 bp or 416 bp double-stranded DNA were obtained. To 30 µl of each amplification product were added 10 µl of formamide and the mixture was heated at 95°C for 10 minutes. The resulting heat-denatured amplification products were poured at a flow rate of 5 µl/min for 10 minutes into measuring cells in which the probe PNA was immobilized as described in Example 1 and resonance signals were measured. For a control, 30 µl of a Tris buffer solution (pH 7.4) was used instead of 30 µl of PCR products. Results are shown in Table 4.

**Table 4**

| Probe | Sample DNA | Amount of PCR product | |
|---|---|---|---|
| | | 0 µl(control) | 30 µl |
| DNA | 143 bp | 3.7 RU | 21.6 RU |
| PNA | 143 bp | 4.4 RU | 211.3 RU |
| PNA | 416 bp | 4.4 RU | 276.2 RU |

As shown in Table 4, even under these conditions for PCR, significant resonance signals were detected with the use of PNA as a probe but signals could hardly be detected with the use of DNA as a probe. These results showed that probe PNAs were more readily hybridized with sample DNAs than probe DNAs.

The 416 bp sample DNA showed larger resonance signals than the 143 bp sample DNA. But in view of the fact that resonance signals increase with an increase in molecular weight of the substance binding with the probe, this resonance signal value was not as great as anticipated. In other words, the 416 bp DNA is not hybridized with the probe PNA as efficiently as the 143 bp DNA. This phenomenon would be due to the greater probability that as its chain length increases, DNA becomes a three-dimensional shape which cannot hybridize with a probe (for example, spherical).

### Example 3

The following four sense primers and one antisense primer were synthesized based on the genome DNA of pathogenic *E. coli* O-157 shown in Figure 4.
Sense primer 1: GCCGGGTTCGTTAATACGGCA (SEQ ID NO. 4) (This sequence is identical to the DNA sequence 301-321 of SEQ ID NO. 1)
Sense primer 2: TTAACCACACCCCACCGGGCA (SEQ ID NO. 7) (This sequence is identical to the DNA sequence 188-208 of SEQ ID NO. 1)
Sense primer 3: TCTCAGGGGACCACATCGGTG (SEQ ID NO. 8) (This sequence is identical to the DNA sequence 160-180 of SEQ ID NO. 1)
Sense primer 4: CGGTATCCTATTCCCGGGAGT (SEQ ID NO. 9) (This sequence is identical to the DNA sequence 53-73 of SEQ ID NO. 1)
Antisense primer 1: GAACGTTCCAGCGCTGCGACA (SEQ ID NO. 6) (This sequence is complementary to the DNA sequence 423-443 of SEQ ID NO. 1)

A solution for PCR having the following components containing these primers and a genome DNA of pathogenic *E. coli* O-157 used as a template was prepared.

**Table 5**

| Component | Concentration |
|---|---|
| Tris buffer solution (pH 8.3) | 25 mM |
| Primer | 1 µM |
| Template DNA | 1% |
| Potassium chloride | 500 mM |
| Magnesium chloride | 15 mM |
| dNTP | 8% |
| Tag polymerase | 0.2% |

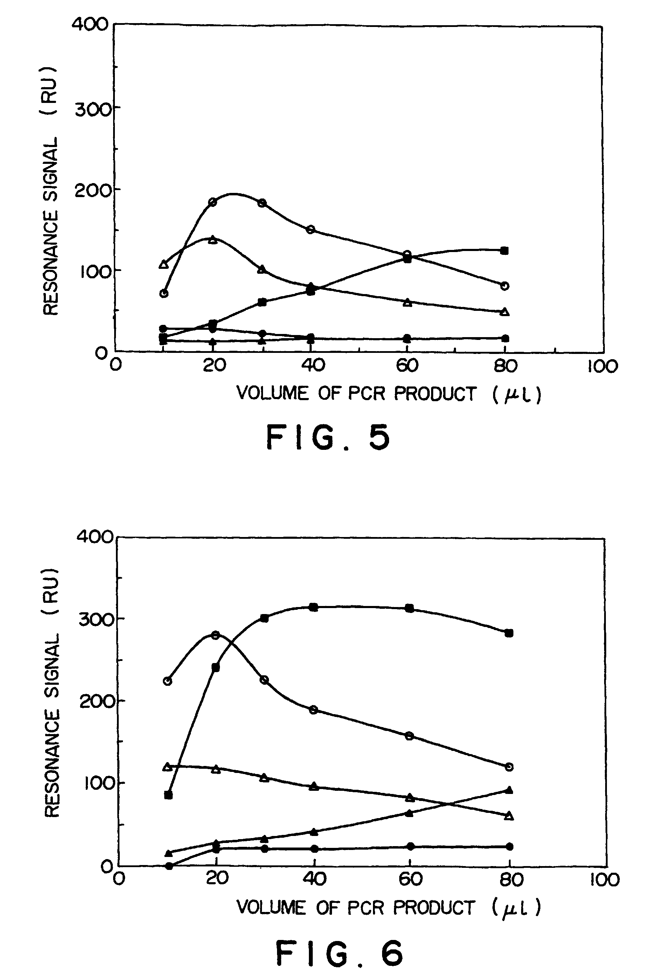

After an initial denaturation (95°C, 3 minutes), PCR was performed for 10 to 40 cycles of denaturation (61°C, 1 minute). annealing (72°C, 1 minute) and elongation (94°C, 1 minute). Four kinds of PCR amplification products containing 143 bp, 256 bp, 284 bp, or 391 bp double-stranded DNA were obtained. To 10 µl, 20 µl, 30 µl, 40 µl, 60 µl or 80 µl of each amplification product were added 10 µl of formamide and the mixture was heated at 95°C for 10 minutes. The resulting heat-denatured amplification products were poured at a flow rate of 5 ml/min for 10 minutes into measuring cells in which the probe PNA was immobilized as described in Example 1 and resonance signals were measured. Results are shown in Figures 5, 6, 7 and 8.

Figure 5 shows the relationship between the amount of PCR products and resonance signals when the length of the sample DNA chain is 143 bp. As shown in the Figure, larger resonance signals were detected as the number of the cycles increased. However, at a higher number of PCR cycles, resonance signals start to diminish when the amount of PCR products exceeds 30 µl.

Figure 6 shows the relationship between the amount of PCR products and resonance signals when the length of the sample DNA chain is 256 bp. Unlike Figure 5, the largest resonance signals were detected for the PCR with 25 cycles.

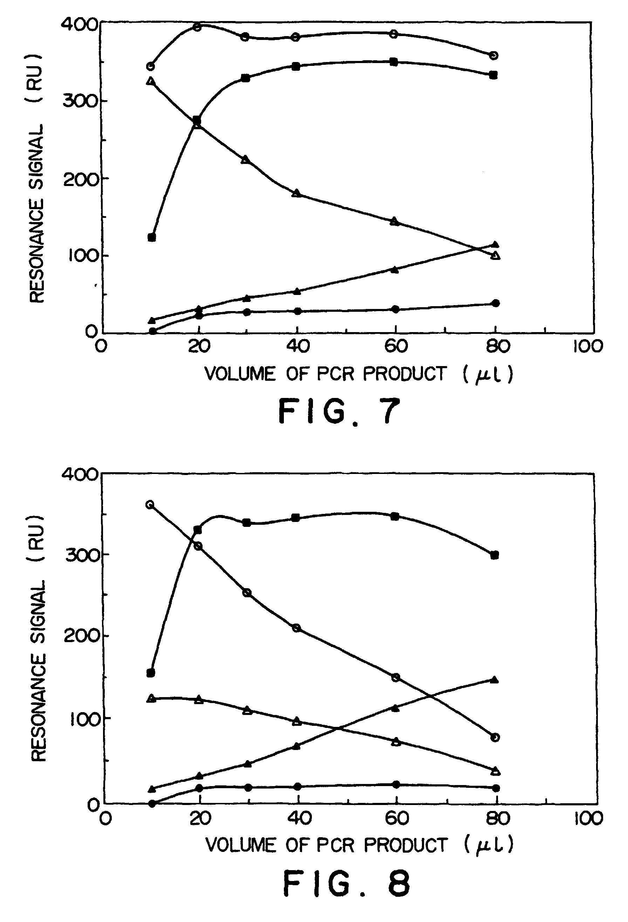

Figure 7 shows the relationship between the amount of amplification products and resonance signals when the length of the sample DNA chain is 284 bp. Resonance signal detection patterns were virtually similar to those in Figure 6.

Figure 8 shows the relationship between the amount of amplification product and resonance signals when the length of the sample DNA chain is 391 bp. Resonance signal detection patterns were virtually similar to those in Figure 6.

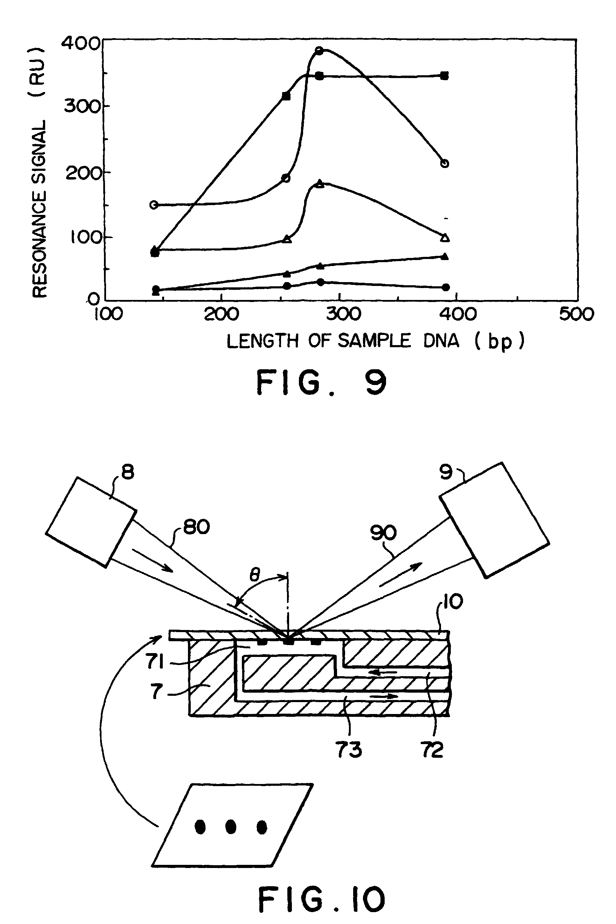

The relationship between DNA chain length and resonance signals was analyzed from the results shown in Figures 5 to 8. The resonance signal values for 40 µl of PCR products were used. Results are shown in Figure 9.

In general, resonance signals increase with an increase in molecular weight of the substance (DNA chain length) binding with the probe, but this is not the case in Figure 9. As mentioned above, this phenomenon would be due to the greater probability that as its chain length increases, DNA becomes a three-dimensional shape which cannot hybridize with a probe.

### Example 4

A layer chrome and then a gold layer were deposited on a 13 mm x 18 mm and 0.3 mm thick blue glass plate (Matsunami Glass Kogyo) by sputtering to produce a measuring chip for a surface plasmon resonance biosensor. Sputtering was carried out at 100 W for 30 seconds to produce a 32.2 angstrom chrome layer; and at 100 W for 150 seconds to produce a 474-angstrom gold layer. This measuring chip was immersed into a 1 mM ethanol solution of 11-mercaptoundecanoic acid for 24 hours to form a thin organic membrane layer on the metal layer. Then, 50 µl of a 5% avidin solution were dropped at 3 spots on the same chip, wherein amides bonds were formed between the avidin molecules and the thin organic membrane molecules, thus immobilizing the avidin molecules.

The following three kinds of PNA with biotin bound at the 5' end (synthesized by Japan Perceptive Inc.). These PNAs have sequences complementary to a part of three kinds of genes, *tdh*l, *tdh*2 and *trh*2, which are the toxic elements of a toxin producing bacteria, *Vibrio parahaemolyticus*.
Sequence A (*tdh*1): AAGTTATTAATCAAT (SEQ ID NO. 10)
Sequence B (*tdh*2): TTTTTATTATATCCG (SEQ ID NO. 11)
Sequence C (*trh*2): CCCAGTTAAGGCAAT (SEQ ID NO. 12)

30 µl of a solution containing the abovementioned PNA (10 µl) were dropped onto the spots where the avidin solution was dropped to immobilize the PNA onto the measuring chip via an avidin-biotin bond.

The measuring chip on which the PNA was immobilized was placed onto a surface plasmon resonance biosensor (SPR-20 type with a modified sensor head and fluid supply and drainage. Denki Kagaku Keiki) (Figure 10). Since the measuring chip of this biosensor can freely move horizontally, resonance signals of the multiple numbers of samples present on the chip can be measured leaving the optical system fixed.

The DNA sequence to be detected was amplified as a fragment of about 300 bp using a PCR (25 cycles). A solution containing the amplified DNA was poured into a measuring cell of the biosensor to measure resonance signals at a flow volume of 10 µl. Results are shown in Table 6.

**Table 6**

| Sequence | A | B | C |
|---|---|---|---|
| Resonance signal (x 10⁻⁴) | 308 (RU) | 298 (RU) | 315 (RU) |

As shown in Table 6, sequences A, B and C all show signals near 300 RU (converted values). Considering the fact that the signals are 10-20 RU (converted values) when no DNA is bound (negative), then it would appear that the DNA was bound to the three kinds of immobilized PNAs (positive).

### Example 5

A metal layer and a thin organic membrane layer were deposited on a blue plate glass and four measuring chips were prepared as described in Example 4. 50 µl of a 5% avidin solution were dropped the avidin molecules.

The following 8 PNAs to which biotin is bound at their 5' ends were synthesized (synthesized by Japan Perceptive Inc.). Sequences A, B and C are PNAs which have sequences complementary to a part of gene *tdh*1, *tdh*2 and *trh*2 of *Vibrio parahaemolyticus*, respectively. Sequences D, E, F, G and H are PNAs which have sequences complementary to 18S rRNA of *Salmonella enteritidis*, a pertussis toxin of *Borderlia pertussis*, *Vibrio cholera* toxin, type I verotoxin of *Escherichia coli* O-157 (pathogenic *E. coli* O-157) and type II verotoxin of *E. coli* O-157, respectively.
Sequence A: AAGTTATTAATCAAT (SEQ ID NO. 10)
Sequence B: TTTTTATTATATCCG (SEQ ID NO. 11)
Sequence C: CCCAGTTAAGGCAAT (SEQ ID NO. 12)
Sequence D: CGCAAACCGTATTAC (SEQ ID NO. 13)
Sequence E: CCAAAGTATTTCCCT (SEQ ID NO. 14)
Sequence F: AATTCGGGTTAATTG (SEQ ID NO. 15)
Sequence G: GGGCGTTATGCCGTA (SEQ ID NO. 16)
Sequence H: TGCAGAGTGGTATAA (SEQ ID NO. 17)

30 µl of a solution containing the PNA (10 µl) were dropped onto the spots where the avidin solution was dropped to immobilize the PNA onto the measuring chip via avidin-biotin bonds.

The measuring chip on which the PNA was immobilized was placed onto the surface plasmon resonance biosensor used in Example 4. The DNA to be detected was amplified using the PCR as described in Example 4. Four DNAs were used; DNAs prepared from *E. coli* O-157, *Vibrio parahaemolyticus*, and *Salmonella* and a combination of DNAs from *E. coli* O-157 and *Salmonella*. A solution containing the amplified DNA was poured into a measuring cell of the biosensor to measure resonance signals at a flow volume of 10 µl. Results are shown in Table 7.

**Table 7**

| | *E. coli* O-157 | *V.parahaemoliticus* | *Salmonella* | *E. coli* O-157 + *Salmonella* |
|---|---|---|---|---|
| Sequence A | 22 | 295 | 10 | 11 |
| Sequence B | 18 | 331 | 12 | 28 |
| Sequence C | 21 | 301 | 18 | 23 |
| Sequence D | 15 | 22 | 321 | 299 |
| Sequence E | 17 | 24 | 22 | 18 |
| Sequence F | 24 | 19 | 33 | 19 |
| Sequence G | 308 | 18 | 24 | 356 |
| Sequence H | 311 | 25 | 26 | 334 |

As shown in Table 7, for the various microorganisms, signals near 300 RU were obtained for positive samples and signals less than 30 RU were obtained for negative samples.

## Claims

1. A method for detecting a target nucleotide sequence, which comprises the steps of:
hybridizing a target nucleotide sequence with a PNA (peptide nucleic acid) which is complementary to the whole or a part of the target nucleotide sequence; and
measuring the degree of hybridization.

2. A method according to claim, which further comprises, prior to the step of hybridizing the target nucleotide sequence with PNA, the step of amplifying the target nucleotide sequence by a polymerase chain reaction.

3. A method according to claim 1 wherein the degree of hybridization is measured by a surface plasmon resonance biosensor.

4. A method according to claim 1 wherein the PNA is immobilized on a measuring chip of a surface plasmon resonance biosensor.

5. A method according to claim 1 wherein two or more target nucleotide sequences are detected.

6. A method according to claim 1 wherein the nucleotide sequence is DNA.
